(19) Europäisches Patentamt
European Patent Office
Office européen des brevets

(11) Publication number: **0 240 964 B1**

# (12) EUROPEAN PATENT SPECIFICATION

(45) Date of publication of patent specification: **15.07.92**

(51) Int. Cl.⁵: **C12Q 1/26**, C12N 9/04

(21) Application number: **87105040.7**

(22) Date of filing: **04.04.87**

## (54) Reagent for the enzymatic determination of primary C1-C4 alcohols and related method.

(30) Priority: **09.04.86 IT 2001286**

(43) Date of publication of application:
**14.10.87 Bulletin 87/42**

(45) Publication of the grant of the patent:
**15.07.92 Bulletin 92/29**

(84) Designated Contracting States:
**AT BE CH DE ES FR GB GR IT LI LU NL SE**

(56) References cited:
**EP-A- 0 019 937**
**EP-A- 0 110 173**
**EP-A- 0 117 032**
**EP-A- 0 121 254**
**EP-A- 0 133 481**
**EP-A- 0 164 008**
**WO-A-85/05127**
**US-A- 4 556 635**

**PATENT ABSTRACTS OF JAPAN, vol. 8, no. 93 (C-220)[1530], 27. April 1984**

(73) Proprietor: **BOEHRINGER MANNHEIM ITALIA S.p.A.**
**Via S. Uguzzone, 5**
**I-20126 Milano(IT)**

(72) Inventor: **Iaccheri, Ennio**
**Via S. Uguzzone, 5**
**I-20126 Milano(IT)**
Inventor: **Manzati, Claudio**
**Via S. Uguzzone, 5**
**I-20126 Milano(IT)**
Inventor: **Prencipe, Lorenzo**
**Via Ginestre, 14**
**I-20095 Cusano Milanino (MI)(IT)**

(74) Representative: **Minoja, Fabrizio**
**Studio Consulenza Brevettuale Via Rossini, 8**
**I-20122 Milano(IT)**

EP 0 240 964 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention refers to a reagent in associated form, for the enzymatic qualitative and/or quantitative determination of $C_1$-$C_4$ primary alcohols and to an analytical method using said reagent complex. There is a urgent need for an analytical method for the determination of primary $C_1$-$C_4$ alcohols, particularly methanol and ethanol, which is sensitive, fast and easy enough to be carried out also by inexperienced personell and without any need of laboratory operations.

Some systems have been therefore developed, based on the reaction with an alcohol oxidase, summarized in the following scheme:

$$RCH_2OH + O_2 \xrightarrow{\text{alcohol oxidase}} R\text{-}CHO + H_2O_2.$$

The formed hydrogen peroxyde is detected by known methods, using a peroxidase and a chromogenic system of the Trinder kind (phenol/4-amino-antipyrine).

EP-A-II0 I73, EP-A-I3348I and EP-A-I64008 disclose test devices wherein the alcohol oxidase, peroxidase and chromogenic reagents are absorbed on suitable carrier, made of hydrophilic material. All these systems require however particular measures, such as the addition of stabilizers or enzyme regulators and the use of specialized procedures in order to overcome the problems connected with the poor stability of alcohol oxidase and its tendence to auto-oxidation with consequent outcome of "false positive" results.

It has now been found that, by using an alcohol oxidase extracted from yeast of the genus Pichia, and a modified Trinder chromogenic reaction wherein the phenol compound is chromotropic acid(4,5-dihydroxy-2,7-naphalenedisulphonic acid) or a salt thereof, it is possible to combine all the three reagents in associated form without any problem of stability.

The reagent complex is in fact indefinitely stable and offers the advantage of not requiring any preliminar operation before use and any addition of stabilizers and particularly serum under analysis would not be diluted offering a more reliable system. No peroxide scavenger (like ascorbic acid or cysteine) is requested since the method of the present invention does not suffer from false positives as it has been demonstrated by analyzing 10 sera ethanol-free whose optical density values were ranging from 0.028 to 0.034.

The absence of said peroxide scavengers allow a sensitivity of 50 mg/100 ml, which is lower than that obtainable according to the prior-art methods.

The peroxidase, which may be of any origin, is used in relevant excess till 100 U.I. per ml with respect to stoichiometric amount: it has been in fact found that peroxidase itself has a stabilizing activity on alcohol oxidase.

As far as the 1-phenyl-pyrazolinone derivative to be used in conjunction with chromotropic acid, 4-amino-antipyrine is particularly preferred, producing a reaction showing an absorption maximum at 590 nm, a region which is convenient for optimal visual analysis.

Other known compounds, such as aminophenazone, propiphenazone, methanpyron, tozalinone, dibupyron, sulphapyrine, may also be used.

According to this invention, it is possible to detect ethanol and other primary $C_1$-$C_4$ alcohols in biological fluids without precipitating the sample, with a linearity range from 50 to 400 mg/dl.

Determination can be effected both at end point and in kinetics, both on a quantitative basis, by means of a colorimetric reading against a standard and semi-quantitatively by means of comparison with a chromatic scale.

This method does not require use of acids for stopping the reaction, can be carried out both manually and in suitable automatic analyzers, and is free from interferences due to pharmaceuticals (e.g. anti-coagulants) or to biological substances that are usually present in biological fluids.

In particular no interference with glucose, uric acid and cholesterol was found, these substances being not a substrate for alcohol oxidase.

The reactive that is object of the present invention can be prepared in a lyophilized form, containing all components and suitable supports for lyophilization such as sugars, aminoacids, polyols, proteins and mixtures thereof. Preferably saccharose, lactose, mannitol, polyvinylpyrrolidone, glycine, glutammic acid and its salt, aspartic acid or its salt, albumine, etc. are employed.

The following examples furtherly describe, but do not limit, the invention.

### EXAMPLE I

**Determination of ethanol by means of a mono-reactive agent prepared in lyophilized form**

Two solutions are prepared, having the following compositions

**Solution "A"**

| | |
|---|---|
| Phosphate buffer pH 7,50 | 500 mMol |
| Triton X 100 | 5 g/lt |

**Solution "B"**

| | |
|---|---|
| Phosphate buffer | 10 mMol |
| Chromotropic acid | 15 mMol |
| 4-amino-antipyrine | 3 mMol |
| EDTA $Na_2$ | 5 mMol |
| Peroxidase | 20,000 U/lt |
| Alchol oxidase | 40,000 U/lt |
| Support for lyophilization | 30 g/lt. |

0,5 ml of solution "A" are dosed in glass vials, they are frozen in a refrigerating cell at -45°C for 4 hours, then, in the same vials, 0,3 ml of solution "B" are dosed.

The product is then lyophilized, avoiding to expose it at a temperature higher than 40°C.

The prepared reaction agent is suitably sealed; after being reconstituted with 3 ml distilled water, it can be used for manual determination, according to the following method:

| | |
|---|---|
| Sample: | serum or plasma |
| Temperature: | 20-25°C or 37°C |
| Wave lenght: | 570-620 nm (585 nm) |
| Cuvette: | l cm optical path |

l0 mcl of sample and l0 mcl of a standard solution containing a known concentration of ethanol (ranging from 50 to 400 mg/dl) are sampled in two different vials of reaction agent reconstituted with 3 ml water each. After shaking, it is necessary to wait at least for 30 minutes at a temperature of 20-25°C or for l5 minutes at 37°C. The absorption of sample solution ($E_c$) and of reference solution ($E_r$) is therefore measured in a suitable spectrophotometer or photocolorimeter at a wavelength of 585 nm, calibrating the instrument by means of a solution consisting only of reconstituted reaction agent.

Calculation: ethanol mg/dl $=\frac{Ec}{Er}$ . conc. ref. solution (mg/dl).

**EXAMPLE 2**

**Automatic determination of ethanol in serum or other biological fluids**

A solution having the following composition is prepared:

| | | |
|---|---|---|
| Phosphate buffer pH 7,50 | 100 | mMol |
| Chromotropic acid | 1,5 | mMol |
| 4-amino-antipyrine | 0,3 | mMol |
| Peroxidase | 2,000 | U/lt |
| Alcohol Oxidase | 2,000 | U/lt |
| Triton X 100 | 1 | g/lt |

The solution is used on automatic analyzers with a sample/reaction agent ratio of l:300.

Reading at 600 nm.

Incubation period: l5 minutes at 37° C or 20-25 at end-point or with determination of Δ E. min. between 2l0 and l000 seconds at 37° C for determination in kinetics.

## EXAMPLE 3

### Manual determination of ethanol in breathed-out air

Reaction agent of Example l, after reconstitution may also be used for the determination of ethanol in breathed out air; it is sufficient that the subject to be tested expires air for a period ranging from l0 and 20 seconds, by means of a cannula, into the reaction agent. The colour formed can be read by means of a spectrophotometer or compared with a chromatic scale.

## EXAMPLE 4

### Determination of alcohol degree in wines or spirits

It is possible to use lyophilized reaction agent of Example I with the same method, but diluting it previously with water l:50 for wines and bitters and l:l00 for brandy and generally all spirits.

| Sample | Value declared | Value found |
| --- | --- | --- |
| Wine C | 13,5% vol. | 13,8% vol. |
| Wine V | 12% vol. | 12,8% vol. |
| Wine T.O. | 12,5% vol. | 12,7% vol. |
| Wine A | 12% vol. | 12% vol. |
| Spirit | 18,5% vol. | 18,7% vol. |
| Wine B | 12% vol. | 11,8% vol. |
| Wine CH | 11,7% vol. | 11,5% vol. |
| Wine G | 10,5% vol. | 11,1% vol. |

**EXAMPLE 5**

I0 samples of serum containing known quantities of ethanol have been analyzed by using the reaction agent prepared in accordance with Example I. Some samples have been previously de-proteinized. The following table evidences how the method of this invention is insensitive to the presence of serum-proteins.

| Samples | Precipitated serum | Not precipitated serum |
|---|---|---|
| 1 | 105,8 mg/dl | 106,1 mg/dl |
| 2 | 53,2 mg/dl | 53,1 mg/dl |
| 3 | 78,9 mg/dl | 78,9 mg/dl |
| 4 | 101,1 mg/dl | 100,9 mg/dl |
| 5 | 43,2 mg/dl | 42,8 mg/dl |
| 6 | 58,6 mg/dl | 58,7 mg/dl |
| 7 | 159,9 mg/dl | 160,0 mg/dl |
| 8 | 65,5 mg/dl | 65,3 mg/dl |
| 9 | 71,3 mg/dl | 71,1 mg/dl |
| 10 | 206,8 mg/dl | 207,0 mg/dl |

## EXAMPLE 6

As in the preceding example, a comparison has been made, between end-point and kinetics determination. Also in this case values obtained on the same samples, but using different techniques , are practically identical and in any case included in the usual experimental error ranges.

| Samples | End-point determination | Kinetics determination |
|---|---|---|
| 1 | 65,9 mg/dl | 65,9 mg/dl |
| 2 | 73,8 mg/dl | 73,7 mg/dl |
| 3 | 105,3 mg/dl | 105,4 mg/dl |
| 4 | 41,8 mg/dl | 42,0 mg/dl |
| 5 | 98,3 mg/dl | 99,1 mg/dl |
| 6 | 125,7 mg/dl | 126,0 mg/dl |
| 7 | 51,1 mg/dl | 50,9 mg/dl |
| 8 | 201,3 mg/dl | 202,1 mg/dl |
| 9 | 157,2 mg/dl | 156,8 mg/dl |
| 10 | 31,9 mg/dl | 32,0 mg/dl |

## EXAMPLE 7

The compounds reported in the following table do not interfere with the determination of ethanol in serum, up to the concentrations shown.

| PHARMACEUTICAL SUBSTANCES | TESTED CONCENTRATION (mg/l) |
|---|---|
| Acetylsalicilic acid | 1000 |
| Ampicilline | 1000 |
| Ascorbic acid | 200 |
| Bezafibrate | 100 |
| Carbochrome | 30 |
| Quinidine | 60 |
| Chloramphenicole | 200 |
| Chlorodiazepoxide | 30 |
| Caffeine | 200 |
| Etaverine | 2 |
| Furosemide | 1750 |
| Glybenclamide | 1 |
| Indomethacin | 100 |
| Metaqualone | 50 |
| Methyldopa | 80 |
| Nicotinic acid | 400 |
| Nitrofurantoin | 18 |
| Metamizol | 200 |
| Ozazepam | 10 |
| Oxytetracycline | 160 |

| | |
|---|---|
| Paracetamol | 200 |
| Phenobarbital | 250 |
| Phenoprocumone | 20 |
| Phenazopyridine | 25 |
| Phenytoin | 200 |
| Probenecid | 1000 |
| Procaine | 2 |
| Pyridamol | 100 |
| Pyritinol (pyritiazine) | 20 |
| Sulphamethoxazole | 600 |
| Theophylline | 200 |
| Trimethoprim | 18 |
| Dexchlorophenir amine | 100 |

| ANTI-COAGULANTS | TESTED CONCENTRATION (mg/dl) |
|---|---|
| Heparin | 20 |
| Sodium Citrate | 1000 |
| Sodium fluoride | 1000 |
| EDTA | 1000 |

| BIOLOGICAL SUBSTANCES | TESTED CONCENTRATION (mg/dl) |
|---|---|
| Glucose | 1000 |
| Fructose | 1000 |
| Galactose | 1000 |
| Bilirubine | 25 |
| Haemoglobin | 1000 |
| Triglycerides | 2010 |

**EXAMPLE 8**

Some experiments of stability have been carried out on various lots of the reaction agent containing lyophilized alcohol oxidase in presence of various carriers. Results are given hereinbelow.

All experiments of lyophilization have been carried out by diluting alcohol oxidase enzymes in a solution of about 35% of saccharose with a phosphate buffer pH 6,60 0,02M.

| Support used | Enzymatic activity | | | | | |
|---|---|---|---|---|---|---|
| | Title before lyophilization | Title after lyophil. | 37°C stressed Kit | | | |
| | | | 7 days | 14 days | 21 days | 28 days |
| -- | 100 | 12 | n.e. | n.e. | n.e. | n.e. |
| 4 A.A. 0,15% | 100 | 85 | 46 | 40 | 30 | 23 |
| POD 15 U/ml | 100 | 92 | 63 | 57 | 51 | 48 |
| 4 A.A 0,15 % + POD 15 U/ml | 100 | 89 | 56 | 49 | 42 | 36 |
| 4 A.A 0,15% + POD 60 U/ml | 100 | 94 | 65 | 62 | 60 | 61 |
| 4 A.A. 0,15% + POD 60 U/ml + Glut. 0,5% | 100 | 95 | 66 | 61 | 63 | 55 |
| 4 A.A. 0,15% + POD 60 U/ml + Glut. 3% | 100 | 102 | 88 | 74 | 72 | 70 |

n.e. = not executed
4 A.A = 4-amino-antipyrine
POD = Peroxidase
Glut. = monobasic sodium glutammate

## Claims

1. Reagent, in associated forms, for the enzymatic determination of primary $C_1$-$C_4$ alcohols comprising an alcohol oxidase extracted from the yeast of the genus Pichia, an excess of peroxidase and chromotropic acid/antipyrine, in the absence of radical scavengers.

2. A reagent according to claim 1, in lyophilized form on inert supports selected in the group consisting of sugars, amminoacids, polyols, proteins or mixtures thereof.

3. A method for the enzymatic determination of primary $C_1$-$C_4$ alcohols by means of alcohol oxidase, peroxidase and chromogenic system characterized by using a reagent consisting of alcohol oxidase extracted from yeast of the genus Pichia, peroxidase in excess and chromotropic acid/antipyrine as a chromogenic system, in associated form.

**Revendications**

1. Réactif, sous forme associée, pour la détermination enzymatique d'alcools primaires en $C_1$-$C_4$, comprenant une alcool oxydase extraite de levure du genre Pichia, un excès de peroxydase, et de l'acide chromotropique/antipyrine, en l'absence de pièges à radicaux.

2. Réactif selon la revendication 1, sous forme lyophilisée sur des supports inertes choisis dans le groupe constitué par les sucres, les acides aminés, les polyols, les protéines ou leurs mélanges.

3. Procédé pour la détermination enzymatique d'alcools primaires en $C_1$-$C_4$ à l'aide d'alcool oxydase, de peroxydase et d'un système chromogène, caractérisé par l'utilisation d'un réactif se composant d'une alcool oxydase extraite de levure du genre Pichia, de peroxydase en excès, et d'acide chromotropique/antipyrine en tant que système chromogène, sous forme associée.

**Patentansprüche**

1. Reagenz, in assoziierter Form, für die enzymatische Bestimmung von primären $C_1$-$C_4$-Alkoholen in Abwesenheit von Radikalfängern, umfassend eine Alkoholoxidase, extrahiert aus Hefe der Gattung Pichia, einen Überschuß an Peroxidase und Chromotropsäure/Antipyrin.

2. Reagenz nach Anspruch 1, in lyophilisierter Form auf inerten Trägern, ausgewählt aus der Gruppe bestehend aus Zuckern, Aminosäuren, Polyolen, Proteinen und Gemischen davon.

3. Verfahren für den enzymatischen Nachweis von primären $C_1$-$C_4$ Alkoholen mittels einer Alkoholoxidase, Peroxidase und eines farbgebenden Systems, gekennzeichnet durch die Verwendung eines Reagenzes bestehend aus Alkoholoxidase, extrahiert aus Hefe der Gattung Pichia, Peroxidase im Überschuß und Chromotropsäure/Antipyrin als farbgebendem System, in assoziierter Form.